# EUROPEAN PATENT SPECIFICATION

(11) **EP 3 527 203 B1**
(45) Date of publication and mention of the grant of the patent: **15.02.2023**
(21) Application number: 17860871.7
(22) Date of filing: 30.04.2017
(51) Int. Cl.: A61K 9/70, A61K 31/4985, A61K 47/38, A61K 47/36, A61K 47/12, A61P 15/10, A61K 9/00

(54) **ORAL DISSOLVABLE FILM OF TADALAFIL AND PREPARATION METHOD THEREFOR**
ORAL LÖSLICHER FILM VON TADALAFIL UND HERSTELLUNGSVERFAHREN DAFÜR
FILM À DISSOLUTION ORALE CONTENANT DU TADALAFIL ET SA MÉTHODE DE PRÉPARATION

(30) Priority: 13.10.2016 CN 201610894411
(43) Date of publication of application: 21.08.2019
(73) Proprietor: Changzhou No.4 Pharmaceutical Factory Co., Ltd, Changzhou, Jiangsu 213018 (CN); JS Innopharm (Shanghai) Ltd., Shanghai 201319 (CN); Wooshin Medics Co. Ltd., Seoul 152-020 (KR)
(72) Inventor: FAN, Xinhua, Changzhou Jiangsu 213018 (CN); TU, Yongrui, Changzhou Jiangsu 213018 (CN); ZHANG, Jintao, Shanghai 201319 (CN); NAM, Tack Soo, Seoul 152-020 (KR); HE, Yun, Changzhou Jiangsu 213018 (CN); ZHOU, Yueyu, Changzhou Jiangsu 213018 (CN); ZHU, Ji, Changzhou Jiangsu 213018 (CN); ZHANG, Mingjie, Changzhou Jiangsu 213018 (CN)
(74) Representative: Danubia Patent & Law Office LLC
(86) International application number: PCT/CN2017/082674
(87) International publication number: WO 2018/068498

(56) References cited:
- EP-A2- 3 295 932
- WO-A1-2015/199380
- WO-A1-2016/094567
- WO-A1-2016/094567
- CN-A- 103 191 075
- CN-A- 104 586 820
- CN-A- 106 389 392
- US-A1- 2006 269 654

## Description

### Field of Invention

The present invention provides a tadalafil medicinal preparation, and particularly provides a tadalafil orally dissolving film and a preparation method thereof, relating to a field of medicinal preparation.

### Description of Related Arts

Erectile dysfunction (ED) is a clinical frequent disease and there are about 150 million ED patients all over the world, which seriously threatens the physical and psychological health of men. More seriously, the ED morbidity shows an increasing trend year by year, and the ED patients are predicted to reach 300 million in 2020. There are many influence factors of ED, which are closely related with the age, mental psychology, angiocarpy, internal secretion, life-style, drugs and injury operation and has a close relationship with the age growth. ED is often accompanied with the chronic disease, such as diabetes, angiosis and adiposis, and is also the incipient alarming symptom of the cardiovascular event. ED as a psychosomatic disease causes the decreased living quality of the patients and partners and brings great psychic pain to the patients, which decreases the self-assessment and self-confidence of the patients and affects the interpersonal relationship of the patients.

World Health Organization (WHO) recommends the oral drug treatment as the first-line treatment method, and the phosphodiesterase 5 (PDE5) inhibitor is the first choice of the oral drug. There are many other treatment methods, such as the intracavernous drug injection treatment, the testosterone supplement therapy, the operative treatment, the stem cell treatment and the gene therapy. Because of the good and lasting effect and the high safety, the PDE5 inhibitor becomes the first-line treatment drug.

PDE widely exists in the organisms, is distributed in the nucleotide metabolic enzyme system of different tissues, and has various biological functions. The PDE family consists of PDE1, PDE2, PDE3, PDE4, PDE5, PDE6, PDE7, PDE8, PDE9, PDE 10 and PDE11. Some PDE can be further divided; for example, PDE1 can be further divided into several subtypes of PDE1a, PDE1b and PDE1c. PDE5 is mainly distributed in the brain, kidney, pancreas, penis and lung. When receiving the sexual stimulus, the parasympathetic nerves, the non-adrenergic non-cholinergic (NANC) nerves and the vascular endothelial cells generate the nitric oxide (NO) under the effect of the nitric oxide synthase (NOS); the NO activates the guanylate cyclase and enables the guanosine triphosphate (GTP) to convert into the cyclic guanosine monophosphate (cGMP); the increase of the cGMP leads to the decrease of the calcium ion concentration in the cytoplasm, resulting in that the smooth muscle becomes loose, the blood flow of the penis increases and the internal pressure of the cavernous body increases; the venous occlusion function of the cavernous body initiates and the penis starts to erect. The PDE5 can degrade the cGMP, so as to weaken the penis.

The PDE5 inhibitor can competitively inhibit the PDE5 through the nitric oxide/cyclic guanosine monophosphate (NO/cGMP) pathway, so as to inhibit the hydrolyzation of the cGMP and increase the cGMP concentration in the smooth muscle cells of the cavernous body of the penis, thereby achieving the treatment effect of the ED.

The available PDE5 inhibitors in the market are sildenafil, vardenafil, tadalafil and avanafil. The sildenafil and the vardenafil have the low selectivity on PDE5 and inhibit the activity of PDE6 distributed on the retina, and the selectivity thereof on PDE5 and PDE6 are far worse than tadalafil, causing the side effects such as the paropsis. In comparison, tadalafil shows a good selectivity on PDE5, while having a relatively low effect on the isozyme such as PDE1, PDE4 and PDE6. The IC50 (half maximal inhibitory concentration) ratio of PDE6 to PDE5 of tadalafil is 780, which is 118 times than that of sildenafil and 269 times than that of vardenafil. Thus, tadalafil has a relatively low inhibition effect on PDE6 distributed on the retina and may not have the side effects such as the paropsis as sildenafil and vardinafil.

American Food and Drug Administration (FDA) approves tadalafil for treating the ED in 2003, and thereafter two more adaption diseases are increased, respectively the benign prostatic hypertrophy and the pulmonary artery hypertension (PAH).

Buccal tadalafil film compositions are disclosed in US 2006/269654 A1 , WO 2016/094567 A1, EP 3 295 932 A2, WO 2015/199380 A1 and EP 3 111 929 A1 .

Conventionally, the available tadalafil preparations in the market in China are mainly tablets, called Cialis. The production specifications of Eli Lilly and Company have 5 mg, 10 mg and 20 mg.

The tablets as the conventional oral solid preparations have been widely accepted by the patients. However, tadalafil has the poor water solubility, and the tadalafil tablet has the quite high requirements on the raw material such as the particle mobility and the particle diameter; especially for the tablet having the small specification of 5 mg, a little deviation during the preparation process will cause the problem of the content uniformity. During the mixing process, because of the effect of mechanical stirring, the local temperature is quite high and is a great test of the raw material stability; moreover, during the in-vivo dissolution process of the tablet, time of the dissolution process will be affected by the prescription and the technology, and different individuals also have the great difference. Thus, how to solve the drug solubility, increase or keep the drug bioavailability and shorten the drug onset time is always the key and difficult problem of the tadalafil preparation.

### SUMMARY OF THE PRESENT INVENTION

An object of the present invention is to provide a tadalafil orally dissolving preparation, namely tadalafil orally dissolving film, for treating erectile dysfunction (ED), so as to solve existing defects in conventional tadalafil preparations. Technical solutions of the present invention are described as follows.

A tadalafil orally dissolving film is prepared by tadalafil, film-forming material, plasticizer, optional correctant, optional colorant and optional aromatic, wherein the film-forming material consists of components of: (a) pullulan; (b) at least one member selected from a group consisting of salicylic acid, tartaric acid, citric acid, oxalic acid and acetic acid; and (c) at least one member selected from a group consisting of hydroxypropyl cellulose, hydroxypropyl methylcellulose and hydroxyethyl cellulose.

Preferably, for the above tadalafil orally dissolving film, the weight percentages of the components (a), (b) and (c) in the film-forming material are respectively 9%-20%, 0.5 %-1 25% and 0.5-9 .25%. Further preferably, the weight percentages of the components (a), (b) and (c) in the film-forming material are respectively 9%, 0.5% and 0.5%.

Preferably, the above tadalafil orally dissolving film comprises following components by weight percentage of: tadalafil: 5%-20%; film-forming material: 10%-30%; plasticizer: 0.1%-5%; aromatic: 0.01%-1%; correctant: 0.01%-1%; colorant: 0.01%-1%; and ethanol solution: 50%-80%.

Preferably, the plasticizer comprises at least one member selected from polyethylene glycol (PEG), glycerinum and triethyl citrate.

Preferably, the correctant comprises at least one member selected from sucralose, aspartame, mannitol, acesulfame potassium and saccharin.

The aromatic is not specifically limited and can be the conventional aromatic pharmaceutic adjuvant in the field, such as orange flavor. The colorant is not specifically limited and can be the conventional colorant pharmaceutic adjuvant in the field, such as blue pigment.

Another object of the present invention is to provide a method for preparing the above tadalafil orally dissolving film, comprising steps of bulk drug treatment, homogeneous emulsification, coating and drying, cutting and roll-dividing, and packaging, wherein:
the step of homogeneous emulsification specifically comprises steps in sequence of: (1) mixing the plasticizer and the ethanol solution, and thereafter processing with the homogeneous emulsification; (2) adding the correctant, and continuing processing with the homogeneous emulsification; (3) adding the tadalafil, and continuing processing with the homogeneous emulsification; (4) adding the film-forming material, and continuing processing with the homogeneous emulsification; and (5) adding the aromatic and the colorant, and continuing processing with the homogeneous emulsification;
the step of bulk drug treatment is to control a particle diameter (D90) of tadalafil bulk drug below 5 µm;
for the above ethanol solution, a volume ratio of ethyl alcohol to water is preferably 1:1-1:4; and
for the above step of homogeneous emulsification, a liquid preparation process is preferred to homogeneously stir and dissolve at a temperature between a room temperature and 30 °C.

Preferably, according to the present invention, the above tadalafil orally dissolving film is prepared through steps of: (1) bulk drug treatment; (2) homogeneous emulsification; (3) coating and drying; (4) cutting and roll-dividing; and (5) packaging, wherein:
the step of bulk drug treatment specifically comprises steps of: processing the tadalafil bulk drug respectively with mechanical pulverization and jet pulverization, and controlling the particle diameter (D90) of the tadalafil bulk drug below 5 µm;
the step of homogeneous emulsification specifically comprises steps of:
   setting a bath temperature to be 35 °C; adding purified water, ethyl alcohol and plasticizer of prescription dose into a homogeneous pot; and homogeneously and uniformly stirring (3000 rpm);
   slowly adding the correctant; setting a homogeneous temperature to be 25 °C and a homogeneous speed to be 3500 rpm; after finishing adding all the correctant, lowering a cover for closing the pot, and continuing homogeneously stirring for 30 minutes; after finishing stirring, verifying whether the correctant is sufficiently dissolved; if not, continuing homogeneously stirring, and processing with vacuum degassing for 20 minutes until the solution is clear;
   rising the cover, and slowly adding the processed tadalafil raw material, wherein the homogeneous speed is 3500 rpm and an addition time is about 30 minutes; lowering the cover, continuing homogeneously stirring for 30 minutes, and observing a dissolving and dispersing condition, wherein a homogeneous time can be appropriate lengthened;
   processing with the vacuum degassing for about 30 minutes; rising the cover, homogeneously and slowly adding the film-forming material, wherein the homogeneous speed is 3500 rpm; after finishing adding, lowering the cover and continuing homogeneously stirring for 1 hour (4000 rpm);
   adding the aromatic and the colorant from an observation window; and according to a mixing and dispersing condition, continuing homogeneously stirring for 1-1.5 hours (4500 rpm); and
   processing with the vacuum degassing manually for 30-60 minutes; slowly stirring, and naturally degassing over the night; and
   after the step of homogeneous emulsification, through coating, drying, cutting, roll-dividing and packaging, the tadalafil orally dissolving film is obtained.

Compared with the conventional tadalafil tablet, the tadalafil orally dissolving film prepared by the present invention has an easy preparation process, a controllable temperature, a good homogeneity, a rapid dissolution in the oral cavity and a high bioavailability.

Moreover, for the tadalafil orally dissolving film prepared by the present invention, the film-forming material consists of the components (a), (b) and (c). Surprisingly, the film-forming material prepared by the above three components enables the micronized tadalafil to disperse in the film-forming material more uniformly, which greatly increases the solubility of the tadalafil, and enables the film to be better formed and to be stronger. According to the observation data of 6 months, it is found that the moisture content is little decreased, which guarantees that the prepared film has a good stability.

**Table 1: Moisture content comparison of tadalafil orally dissolving films prepared by different form-filming matrixes**

| Film-forming matrix | Moisture content (%) | |
|---|---|---|
| | 0 month | 6 months |
| Hydroxyalkyl cellulose+povidone | 7.5 | 3.8 |
| Linear-chain polysaccharide+hydroxyalkyl cellulose | 7.3 | 4.2 |
| Linear-chain polysaccharide+hydroxyalkyl cellulose+organic acid | 7.5 | 7.1 |

Moreover, because of the low solubility of tadalafil, in many preparations, in order to increase the dissolution rate, the surfactant is often added. For example, according to the Chinese patent application, CN 201310153588.X, in order to increase the dissolution rate, the lauryl sodium sulfate is added in the prescription. However, the surfactant generally has skin and mucosa irritation. In the oral preparations, such as the chewable tablet and the orally dissolving film, the addition of the surfactant will greatly affect the taste, and the patients have the poor compliance. However, the tadalafil orally dissolving film provided by the present invention not only has the high dissolution rate, but also avoids the use of the surfactant. The film-forming material prepared by the film-forming matrix consisting of three specific components covers the micronized tadalafil and greatly increases the dissolution rate. Through the dissolution experiments, the rapid dissolution property is also proved.

Moreover, according to the present invention, through the treatment of the raw material, the particle diameter of the raw material is controlled in an appropriate range. Through the solid dispersion technology, the dispersion uniformity of the raw material in the liquid is improved, so that the content uniformity is guaranteed and meanwhile the dissolution rate of the drug is increased. Compared with the other tadalafil tablets, the tadalafil orally dissolving film provided by the present invention dissolves in the oral cavity under the effect of the saliva and enters the stomach with swallowing of the saliva, while the conventional tablets dissolve after entering the stomach. The rapid dissolution can greatly increase the dissolution rate of the drug. Moreover, compared with the tablets, the orally dissolving film provided by the present invention is convenient to carry and take, has the better compliance and can easily take without water.

### BRIEF DESCRIPTION OF THE DRAWINGS

Fig. 1 is a comparison diagram of dissolution curves in pH1.2 according to a first example of the present invention.
Fig. 2 is a comparison diagram of dissolution curves in pH4.0 according to the first example of the present invention.
Fig. 3 is a comparison diagram of dissolution curves in pH6.8 according to the first example of the present invention.
Fig. 4 is a comparison diagram of dissolution curves in water according to the first example of the present invention.

### DETAILED DESCRIPTION OF THE PREFERRED EMBODIMENT

In order to better illustrate a tadalafil orally dissolving film and a preparation method thereof provided by the present invention, examples of the present invention are described as follows.

### Example 1

### Prescription:

| | | |
|---|---|---|
| | Tadalafil | 20g |
| Film-forming material | Pullulan | 36g |
| | Hydroxypropyl methylcellulose | 2g |
| | Salicylic acid | 2g |
| Plasticizer | Polyethylene glycol | 0.4g |
| Correctant | Mannitol | 20g |
| | Sucralose | 2g |
| Aromatic | Orange flavor | 0.04g |
| Colorant | Blue pigment | 2g |
| Solvent | Ethyl alcohol | 66g |
| | Purified water | 249.56g |
| | | 400g |

### Preparation method:

### ① Bulk drug treatment

Processing tadalafil bulk drug respectively with mechanical pulverization and jet pulverization, and controlling a particle diameter (D90) of the tadalafil bulk drug below 5 µm.

### (2) Homogeneous emulsification

Setting a bath temperature to be 35 °C; adding 249.56g purified water, 66g ethyl alcohol and 0.4g plasticizer of prescription dose into a homogeneous pot; and homogeneously and uniformly stirring (3000 rpm);
slowly adding 22g correctant; setting a homogeneous temperature to be 25 °C and a homogeneous speed to be 3500 rpm; after finishing adding all the correctant, lowering a cover for closing the pot, and continuing homogeneously stirring for 30 minutes; after finishing stirring, verifying whether the correctant is sufficiently dissolved; if not, continuing homogeneously stirring, and processing with vacuum degassing for 20 minutes until solution is clear;
rising the cover, and slowly adding the processed tadalafil raw material, wherein the homogeneous speed is 3500 rpm and an addition time is about 30 minutes; lowering the cover, continuing homogeneously stirring for 30 minutes, and observing a dissolving and dispersing condition, wherein a homogeneous time can be appropriate lengthened;
processing with the vacuum degassing for about 30 minutes; rising the cover, homogeneously and slowly adding the film-forming material (40g pullulan and 2g hydroxypropyl methylcellulose), wherein the homogeneous speed is 3500 rpm; after finishing adding, lowering the cover and continuing homogeneously stirring for 1 hour (4000 rpm);
adding 0.04g aromatic and 2g colorant from an observation window; and according to a mixing and dispersing condition, continuing homogeneously stirring for 1-1.5 hours (4500 rpm); and
processing with the vacuum degassing manually for 30-60 minutes; slowly stirring, and naturally degassing over the night.

### ③ Coating and drying

Setting a coating thickness to be 600-650 µm, an operation speed to be 25 cm/min, and a drying temperature to be 50-55 °C; and uniformly coating drug suspension liquid on a backing film.

### ④ Cutting and roll-dividing

Setting a cutting width to be 30 mm; and dividing a coated drug roll into several small rolls having a width of 30 mm.

### ⑤ Packaging (5 mg/sheet)

Setting a die-cutting length to be 25 mm/sheet and a packaging speed to be 1200 sheet/min; die-cutting and packaging the small roll into small sheets having a size of 30 mm*25 mm one by one.

### Example 2

### Prescription:

| | | |
|---|---|---|
| | Tadalafil | 80g |
| Film-forming material | Pullulan | 80g |
| | Hydroxypropyl cellulose | 37g |
| | Tartaric acid | 3g |
| Plasticizer | Triethyl citrate | 20g |
| Correctant | Mannitol | 35g |
| | Aspartame | 5g |
| Aromatic | Strawberry flavor | 4g |
| Colorant | Blue pigment | 0.04g |
| Solvent | Ethyl alcohol | 67.98g |
| | Purified water | 67.98g |
| | | 400g |

### Preparation method:

Preparing according to the method illustrated in example 1, wherein a packaging specification is 20 mg/sheet.

### Example 3

### Prescription:

| | | |
|---|---|---|
| | Tadalafil | 40g |
| Film-forming material | Pullulan | 60g |
| | Hydroxyethyl cellulose | 15g |
| | Citric acid | 5g |
| Plasticizer | Glycerinum | 20g |
| Correctant | Acesulfame potassium | 0.04g |
| Aromatic | Orange flavor | 0.04g |
| Colorant | Blue pigment | 4g |
| Solvent | Ethyl alcohol | 63.98g |
| | Purified water | 191.94g |
| | | 400g |

### Preparation method:

Preparing according to the method illustrated in example 1, wherein a packaging specification is 20 mg/sheet.

### Example 4

### Prescription:

| | | |
|---|---|---|
| | Tadalafil | 20g |
| Film-forming material | Pullulan | 60g |
| | Hydroxypropyl cellulose | 18g |
| | Oxalic acid | 2g |
| Plasticizer | Polyethylene glycol | 5g |
| Correctant | Saccharin | 0.1g |
| Aromatic | Orange flavor | 0.04g |
| Colorant | Blue pigment | 2g |
| Solvent | Ethyl alcohol | 58.57g |
| | Purified water | 234.29g |
| | | 400g |

### Preparation method:

Preparing according to the preparation method illustrated in example 1, wherein a packaging specification is 5 mg/sheet.

### Example 5

### Prescription:

| | | |
|---|---|---|
| | Tadalafil | 40g |
| Film-forming material | Pullulan | 50g |
| | Hydroxypropyl methylcellulose | 8g |
| | Acetic acid | 2g |
| Plasticizer | Triethyl citrate | 2g |
| Correctant | Mannitol | 19g |
| | Aspartame | 1g |
| Aromatic | Lemon flavor | 1g |
| Colorant | Blue pigment | 2g |
| Solvent | Ethyl alcohol | 75g |
| | Purified water | 200g |
| | | 400g |

### Preparation method:

Preparing according to the preparation method illustrated in example 1, wherein a packaging specification is 20 mg/sheet.

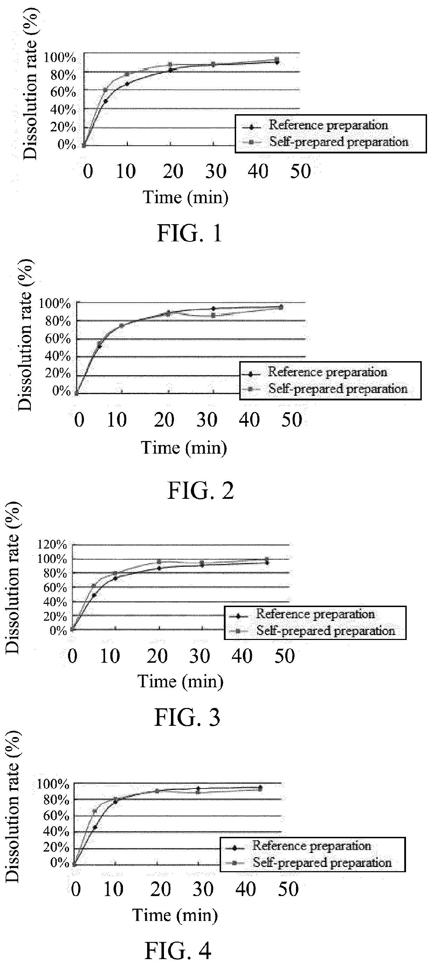

According to the present invention, the tadalafil orally dissolving film is prepared through a suspension liquid coating method. The production process is stable and controllable; a production scale of above 10 thousand sheets is achieved; and the stability of the samples which are continuously produced meets the requirements. In order to analyze the dissolution acts of the tadalafil orally dissolving film provided by the present invention and the other available tadalafil preparation in the market (the tadalafil tablet, Cialis) as the reference preparation in different pH conditions, three batches of the tadalafil orally dissolving film (the tested preparation) which are continuously prepared according to the example 1 and the reference preparation are tested respectively in the pH1.2 condition with 0.5% of lauryl sodium sulfate, the pH4.5 condition, the pH7.5 condition and the water condition, and the dissolution curves thereof are showed in Figs. 1-4. It is showed by the in-vitro release test that the release rate is above 80% in 10 minutes and is above 90% in 30 minutes, which has the consistent dissolution act as the conventional taladafil tablet. According to the human bioequivalence test abroad and the animal in-vivo pharmacokinetics test in China, it is showed that the tadalafil orally dissolving film provided by the present invention has the bioequivalence with the conventional tadalafil tablet, which meets the requirements of drug production and registration.

**Table 2: Research results of human bioequivalence test abroad**

| BE | Kinetic parameter | Results | 90% confidence interval |
|---|---|---|---|
| Tested preparation/reference preparation | AUC | 1.095 | 101.1%∼117.9% |
| | Cmax | 1.088 | 92.4%∼123.3% |

**Table 3: Research results of animal in-vivo pharmacokinetics test**

| BE | Kinetic parameter | 90% confidence interval |
|---|---|---|
| Tested preparation/reference preparation | AUC | 85.7%∼108.3% |
| | Cmax | 88.8%∼122.5% |

Three batches of tadalafil orally dissolving film which are continuously prepared according to the example 1 stay at a condition with a temperature of 30 °C and a relative humidity of 65% for 6 months, the appearance, content, related material and dissolution rate thereof are observed, and the results thereof are showed in Table 4.

**Table 4: Results of stability test**

| Product | | Batch 1 | Batch 2 | Batch 3 |
|---|---|---|---|---|
| Appearance | | Light blue smooth film | Light blue smooth film | Light blue smooth film |
| Content | | 98.67% | 99.82% | 99.48% |
| Related material | | 0.03% | 0.02% | 0.03% |
| Dissolution rate | 10 minutes | 84.3% | 84.1% | 83.2% |
| | 30minutes | 94.5% | 93.3% | 93.4% |

## Claims

1. A tadalafil orally dissolving film, which is prepared by tadalafil, film-forming material, plasticizer, optional correctant, optional colorant and optional aromatic, wherein the film-forming material consisting of components of: (a) pullulan; (b) at least one member selected from a group consisting of salicylic acid, tartaric acid, citric acid, oxalic acid and acetic acid; and (c) at least one member selected from a group consisting of hydroxypropyl cellulose, hydroxypropyl methylcellulose and hydroxyethyl cellulose.

2. The tadalafil orally dissolving film, as recited in claim 1, wherein: the plasticizer comprises at least one member selected from polyethylene glycol (PEG), glycerinum and triethyl citrate.

3. The tadalafil orally dissolving film, as recited in any one of claims 1-2, wherein: weight percentages of the components (a), (b) and (c) in the film-forming material are respectively 9%-20%, 0.5%-1.25% and 0.5-9.25%.

4. The tadalafil orally dissolving film, as recited in any one of claims 1-3, wherein: the tadalafil orally dissolving film comprises components by weight percentage of: tadalafil: 5%-20%; film-forming material: 10%-30%; plasticizer: 0.1%-5%; aromatic: 0.01%-1%; correctant: 0.01%-1%; colorant: 0.01%-1%; and ethanol solution: 50%-80%.

5. A method for preparing the tadalafil orally dissolving film as recited in any one of claims 1-4, comprising steps of bulk drug treatment, homogeneous emulsification, coating and drying, cutting and roll-dividing, and packaging, wherein:
the step of homogeneous emulsification specifically comprises steps in sequence of: (1) mixing the plasticizer and the ethanol solution, and thereafter processing with the homogeneous emulsification; (2) adding the correctant, and continuing processing with the homogeneous emulsification; (3) adding the tadalafil, and continuing processing with the homogeneous emulsification; (4) adding the film-forming material, and continuing processing with the homogeneous emulsification; and (5) adding the aromatic and the colorant, and continuing processing with the homogeneous emulsification.

6. The method as recited in claim 5, wherein: the step of bulk drug treatment is to control a particle diameter (D90) of tadalafil bulk drug below 5 µm.

7. The method as recited in claim 5, wherein a volume ratio of ethyl alcohol to water in the ethanol solution is 1:1-1:4.

## Patentansprüche

1. Ein Tadalafil oral auflösender Film, hergestellt aus Tadalafil, filmbildendem Material, Weichmacher, gegebenenfalls Korrektor, gegebenenfalls Farbstoff und gegebenenfalls Aromastoff, wobei das filmbildende Material die folgenden Komponenten enthält: (a) Pullulan; (b) mindestens ein Mitglied gewählt aus der Gruppe enthaltend Salizylsäure, Weinsäure, Zitronensäure, Oxalsäure und Essigsäure; und (c) mindestens ein Mitglied gewählt aus der Gruppe enthaltend Hydroxypropyl-Zellulose, Hydroxypropylmethyl-Zellulose und Hydroxyethyl-Zellulose.

2. Der Tadalafil oral auflösende Film, wie im Anspruch 1 angegeben, wobei: der Weichmacher mindestens ein Mitglied gewählt aus der Gruppe enthaltend Polyethylen-glykol (PEG), Glyzerinum und Triethyl-citrat enthält.

3. Der Tadalafil oral auflösende Film, wie in einem der Ansprüche 1-2 angegeben, wobei: Gewichtsprozente der Komponente (a), (b) und (c) im filmbildenden Material jeweils 9%-20%, 0,5%-1,25% und 0,5-9,25% beträgt.

4. Der Tadalafil oral auflösende Film, wie in einem der Ansprüche 1-3 angegeben, wobei: der Tadalafil oral auflösende Film, im Gewichtsprozent angegeben, die folgenden Komponente enthält: Tadalafil: 5%-20%; filmbildendes Material: 10%-30%; Weichmacher: 0,1%-5%; Aromastoff: 0,01%-1%; Korrektor: 0,01%-1%; Farbstoff: 0,01%-1%; und Ethanol-Lösung: 50%-80%.

5. Ein Verfahren zur Herstellung vom Tadalafil oral auflösenden Film, wie in einem der Ansprüche 1-4 angegeben, enthaltend die Schritte von Bulk-Wirkstoffbehandlung, homogener Emulgierung, Beschichtung und Trocknung, Schneiden und Rollenteilung, und Verpackung, wobei:
der Schritt von homogener Emulgierung insbesondere Schritte in folgender Reihenfolge enthält: (1) Mischung den Weichmacher und die Ethanol-Lösung, und dann Verarbeitung mit der homogenen Emulgierung; (2) Zugabe vom Korrektor, und Weiterverarbeitung mit der homogenen Emulgierung; (3) Zugabe vom Tadalafil, und Weiterverarbeitung mit der homogenen Emulgierung; (4) Zugabe vom filmbildenden Material, und Weiterverarbeitung mit der homogenen Emulgierung; und (5) Zugabe von Aromastoff und Farbstoff, und Weiterverarbeitung mit der homogenen Emulgierung.

6. Das Verfahren wie im Anspruch 5 angegeben, wobei: der Schritt von Bulk-Wirkstoffbehandlung zu kontrollieren des Teilchendurchmessers (D90) vom Tadalafil Bulk-Wirkstoff unter 5 µm ist.

7. Das Verfahren, wie im Anspruch 5 angegeben, wobei das Volumenverhältnis von Ethylalkohol zum Wasser in der Ethanol-Lösung 1: 1-1:4 beträgt.

## Revendications

1. Film à dissolution orale de tadalafil, qui est préparé par du tadalafil, un matériau filmogène, un plastifiant, un correcteur facultatif, un colorant facultatif et un aromatique facultatif, dans lequel le matériau filmogène est constitué des composants de: (a) pullulan; (b) au moins un membre choisi du group constitué de : acide salicylique, acide tartrique, acide citrique, acide oxalique et acide acétique; et (c) au moins un membre choisi du group constitué de hydroxypropyl cellulose, hydroxypropyl méthylcellulose et hydroxyéthyl cellulose.

2. Le film à dissolution orale de tadalafil selon la revendication 1, dans lequel le plastifiant: comprend au moins un membre choisi parmi du polyéthylène glycol (PEG), de la glycérine et du citrate de triéthyle.

3. Le film à dissolution orale de tadalafil selon l'une quelconque des revendications 1 à 2, dans lequel: les pourcentages en poids des composants (a), (b) et (c) dans le matériau filmogène sont respectivement 9%-20%, 0,5%-1,25% et 0,5-9,25%.

4. Le film à dissolution orale de tadalafil selon l'une quelconque des revendications 1 à 3, dans lequel: le film à dissolution orale de tadalafil comprend des composants par pourcentage en poids de : tadalafil: 5%-20%; matériau filmogène: 10%-30%; plastifiant: 0,1%-5%; aromatique: 0,01%-1%; correcteur: 0,01%-1%; colorant: 0,01%-1%; et solution d'éthanol: 50%-80%.

5. Une méthode de préparation du film à dissolution orale de tadalafil selon l'une quelconque des revendications 1 à 4, comprenant les étapes de traitement de médicament en vrac, d'émulsification homogène, d'enrobage et séchage, de découpage et division en rouleau, et d'emballage, dans lequel:
l'étape d'émulsification homogène comprend spécifiquement les étapes en séquence de: (1) mélanger le plastifiant et la solution d'éthanol, et puis traiter avec l'émulsification homogène; (2) ajouter le correcteur, et continuer de traiter avec l'émulsification homogène; (3) ajouter le tadalafil, et continuer de traiter avec l'émulsification homogène; (4) ajouter le matériau filmogène, et continuer de traiter avec l'émulsification homogène; et (5) ajouter l'aromatique et le colorant, et continuer de traiter avec l'émulsification homogène.

6. La méthode selon la revendication 5, dans laquelle l'étape de traitement de médicament en vrac sert à contrôler le diamètre de particule (D90) de tadalafil en vrac inférieur à 5 µm.

7. La méthode selon la revendication 5, dans laquelle le rapport volumique de l'alcool éthylique à l'eau dans d'éthanol est de 1:1-1:4.
